# EUROPEAN PATENT APPLICATION

(11) **EP 1 683 560 A1**
(43) Date of publication of application: **26.07.2006**
(21) Application number: 04745323.8
(22) Date of filing: 26.05.2004
(51) Int. Cl.: B01D 9/02, A61K 47/26, A61K 47/10

(54) **RADIAL SPHERICAL CRYSTALLIZATION PRODUCT, PROCESS FOR PRODUCING THE SAME AND DRY POWDER PREPARATION CONTAINING THE CRYSTALLIZATION PRODUCT**

(30) Priority: 10.06.2003 JP 2003165565
(71) Applicant: Taisho Pharmaceutical Co. Ltd., Tokyo 170-8633 (JP)
(72) Inventor: DANJO, Kazumi, c/o Meijyo University, Aichi 4688503 (JP); OKAMOTO, Hirokazu, c/o Meijyo University, Aichi 4688503 (JP); FURUDATE, Takeaki, TAISHO PHARMACEUTICAL CO., LTD., Tokyo 1708633 (JP)
(74) Representative: HOFFMANN EITLE
(86) International application number: PCT/JP2004/007171
(87) International publication number: WO 2004/110585

(57) **Abstract**

A radial spherical crystallization product having multiple needle-shaped projections radially extending outward from the core portion. The radial spherical crystallization product is produced by, for example, bringing a supercritical fluid mixed with a modifier according to necessity and a solution containing a sample component, which have been introduced through different flow channels, into contact with each other at the time of emission from the flow channels into a crystallization vessel.

This radial spherical crystallization product can be used as a drug delivery carrier or medicine for transmucous or transpulmonary administration or as a fine carrier or fine stock drug such as dry powder inhaler (DPI).

## Description

### TECHNICAL FIELD

The present invention relates to a radial spherical crystallization product, and more particularly to a radial spherical crystallization product with needle-shaped projections produced by a crystallization technology using a supercritical fluid. The radial spherical crystallization product can be used as a drug delivery carrier for transmucous or transpulmonary administration.

### BACKGROUND ART

A dry powder inhaler (DPI) comprises a raw drug powder which is inhaled and delivered for administration to the pulmonary alveoli or bronchial tubes of the patient. For inhalation, the particle diameter of the raw drug in the dry powder inhalant must be in the range of 0.5-5 µm. Conventionally, the raw drug is crushed into particles having a diameter in this range. In this instance, adhesion and cohesiveness of the particles due to static electricity have to be taken into consideration, because crushing of raw drugs causes problems such as particle adhesion to the inhalant device and formation of secondary particles resulting from particle aggregation.

To overcome these problems, a method of causing fine particles of raw drug to adhere to a carrier (a carrier method) with an objective of increasing the rate of particle emission from the device and to prevent particle aggregation was developed. However, if the carriers to which the raw drugs adhere on the surface thereof are filled into the device and inhaled, a turbulent flow occurring in the device during inhalation causes the raw drugs to dissociate from the carriers. Although some of the raw drugs reach the target region, the carriers may remain in the mouth. For this reason, the inhalation efficiency (lung transport factor) in this method is as low as 30% and the remaining 70% of the inhalant adheres to regions other than the target, which may lead to side effects.

Recently, many documents concerning supercritical fluids and methods for their use have been published (Jasco Report "Supercritical Technology", Jasco Inc., May 8, 1997). Supercritical fluids are fluids that exist in a state above the critical temperature (Tc) and critical pressure (Pc). The following are known characteristics of supercritical fluids.
(1) Supercritical fluids have a diffusion coefficient larger than normal liquid, and lower viscosity and surface tension.
(2) Due to high compressability as compared with ideal gases, the fluid density greatly changes according to a slight change in pressure, whereby the solvation power can be freely controlled.
   Generally, the density of a supercritical fluid under ordinary conditions is 0.1-0.9 g·dm⁻³.
(3) Many supercritical fluids are gases under atmospheric pressure and temperature, and therefore do not require evaporation-concentration steps essential to conventional liquid extraction methods.
(4) Many supercritical fluids produce an atmosphere that does not oxidize, specifically, does not degrade thermally unstable compounds due to their inertness and temperature under ordinary conditions.

Carbon dioxide is most commonly used as a supercritical fluid, because carbon dioxide is cheap and non-toxic, does not bum, and has a low critical temperature, whereby a supercritical state can be easily reached.

Based on these physical properties, technologies for extracting and forming particles using supercritical fluids have been developed. Among them, two methods for forming particles are particularly noteworthy.

One of the methods, rapid expansion of supercritical solution (RESS), comprises dissolving a target solute in a supercritical fluid and rapidly expanding the supercritical fluid to atmospheric pressure in order to crystallize particles (Peter York, "Strategies for particle using supercritical fluid technologies", Pharmaceutical Science & Technology Today, Vol. 2, No. 11, pp. 425-467, 1999).

The other method, gas antisolvent (GAS) recrystallization method, is useful when the target solid does not dissolve in the supercritical fluid or modified supercritical fluid or when the target solid possesses only a very low solubility. In this method, the target solute is dissolved in a common solvent. A supercritical fluid of carbon dioxide or the like is introduced into the solution to cause the volume of the solution to expand rapidly. As a result, the solvation power rapidly decreases in a short period of time thereby providing an opportunity for the particles to crystallize (Peter York, "Strategies for particle using supercritical fluid technologies", Pharmaceutical Science & Technology Today, Vol. 2, No. 11, pp. 425-467, 1999).

However, there have been no reports disclosing the use of crystallization technology using a supercritical fluid in the preparation of a dry powder carrier for transmucous or transpulmonary administration.

### DISCLOSURE OF THE INVENTION

### PROBLEMS TO BE SOLVED BY THE INVENTION

In view of the above, the object of the present invention is to provide a fine particle raw drug or a fine particle carrier useful as a drug or a drug delivery carrier in transmucous or transpulmonary administration such as DPI, wherein the raw drug itself or the raw drug adhering to the carrier is capable of successfully reaching the target regions of the lungs and bronchial tubes.

### MEANS FOR SOLVING THE PROBLEMS

As a result of intensive research to obtain a fine particle raw drug or fine particle carrier suitable for dry powder inhaler, the present inventors discovered that a fine radial spherical crystallization product possessing needle-shaped projections radially extending from the core can be obtained by adjusting conditions during crystallization using a supercritical fluid.

A first embodiment of the present invention is to provide a radial spherical crystallization product comprising needle-shaped projections radially extending from the core.

A second embodiment of the present invention is to provide a radial spherical crystallization product obtained by emitting a supercritical fluid or a mixture of a supercritical fluid and a modifier and a solution comprising a sample component into a crystallization vessel through different flow channels, thereby causing them to come in contact with each other as they are emitted into the crystallization vessel.

A third embodiment of the present invention is to provide a method for preparing a radial spherical crystallization product comprising emitting a supercritical fluid or a mixture of a supercritical fluid and a modifier and a solution comprising a sample component into a crystallization vessel through different flow channels, thereby causing them to come in contact with each other as they are emitted into the crystallization vessel.

A fourth embodiment of the present invention is to provide a dry powder preparation comprising, as an active ingredient, a radial spherical crystallization product prepared using a pharmaceutical drug as a sample component.

A fifth embodiment of the present invention is to provide a dry powder preparation comprising a radial spherical crystallization product as a drug carrier, prepared using a drug carrier material as a sample component.

### EFFECT OF THE INVENTION

A novel fine radial spherical crystallization product can be obtained in the present invention. The radial spherical crystallization product formed from a drug substance can be used as a raw drug for a DPI and the radial spherical crystallization product formed from a drug carrier can be used as a DPI carrier for delivering a drug to the lungs or mucosa.

### BEST MODE FOR CARRYING OUT THE PRESENT INVENTION

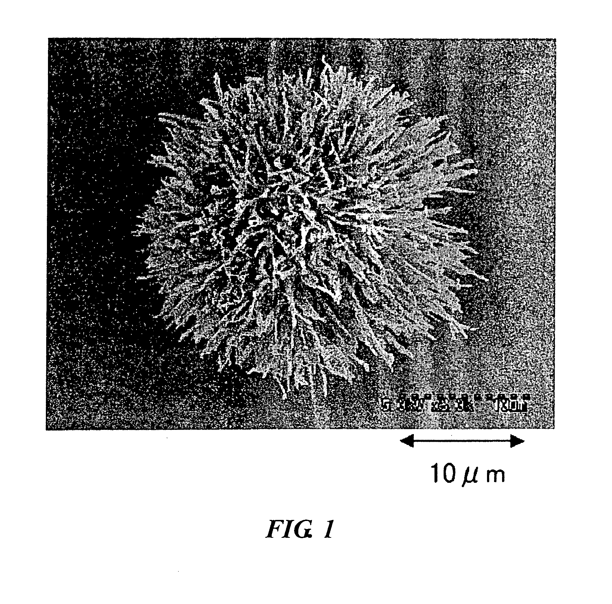

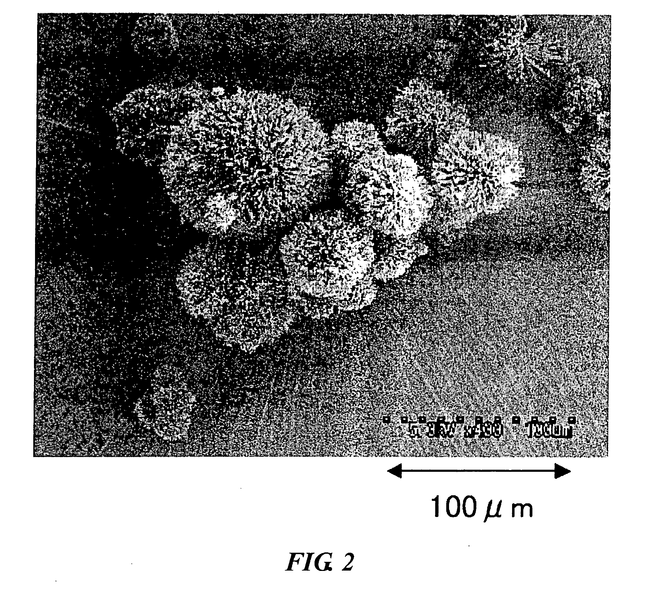

The radial spherical crystallization product of the present invention is a crystallization product comprising needle-shaped projections radially extending from the core to form a spherical configuration (refer to Figs. 1 and 2). Other ways to describe this configuration include reference to an echinoid, chestnut bur, or spherical moss (Cladophora sauteri) covered with a spherical shell with long thorns projecting therefrom.

The crystallization product with this shape is obtained by forming crystals of a sample component in a supercritical fluid whereby crystals with needle-shaped projections radially extending from the core of the component can be obtained.

The number and shape of the needle-shaped projections of the radial spherical crystallization product of the present invention differ according to the crystallization conditions. The term "needle-shaped" in the present invention includes cylindrical and plate-like shapes. The term "spherical shape" includes a completely spherical shape, a nearly spherical shape, an elliptical spherical shape, and a planular spherical shape.

The aerodynamic diameter of the radial spherical crystallization product (hereinafter referred to as "crystallization product") of the present invention is about 0.1-20 µm. When used in transpulmonary administration, for example, the diameter is preferably 0.1-5 µm. When used in local administration to the bronchial tubes and the like, the diameter is preferably 0.5-20 µm.

The aerodynamic diameter refers not to the geometrical length, but to the particle diameter in relation to inertia during air flow, and the particle size distribution thereof can be measured using, for example, an Andersen cascade impactor, multi-stage liquid impinger, and the like. As a method for easy measurement, an Aerosizer and the like can be given.

The bulk density of the crystallization product is about 100 mg/ml or less and preferably 30-100 mg/ml.

The bulk density is obtained by filling a container having a known volume with a powder using a specified method and dividing the weight of the powder by the volume of the container including the void space between the powder particles. For example, a 10 ml graduated cylinder is carefully filled with a sample component, and the weight of the sample component filled up to the 10 ml marking is divided by 10 ml.

The above crystallization product of the present invention is prepared by, for example, emitting a crystallization vessel with a supercritical fluid comprising a modifier when necessary and a solution comprising a sample component through different flow channels, thereby causing them to come into contact with each other as they are emitted into the crystallization vessel.

In the method for preparing the above crystallization product, the "sample component" is a substance to be crystallized, which is insoluble or scarcely soluble in the later-mentioned supercritical fluid or a mixture of the supercritical fluid with a modifier. As long as the substance has the above properties there are no restrictions to the substance used as the component. When the radial spherical crystallization product of the present invention is used as a drug or as a drug carrier, the substance is preferably a pharmaceutically approved active ingredient, an excipient, or a combination of the two.

A crystallization product prepared from a sample component comprising an active ingredient can be used as is in transmucous or transpulmonary administration. When a crystallization product is prepared using an excipient as a sample component, the crystallization product can be used in transmucous and transpulmonary administration by supporting an active ingredient on the needle-shaped projections. When the crystallization product is used as a dry powder carrier, the sample component preferably possesses good biocompatibility, for example, sugar or sugar alcohol. Lactose can be given as a typical sugar.

The "solution comprising a sample component" in the present invention is a solution comprising a sample component to be crystallized either dissolved or suspended therein. The solvent is appropriately selected according to the type of sample component to be crystallized. For example, if the sample component is sugar or sugar alcohol, water is preferably used. A mixture of two or more types of solvents may be used.

The "supercritical fluid" used in the production of the crystallization product indicates a fluid in a state above a critical pressure (Pc) and critical temperature (Tc). Most supercritical fluids exist at a pressure within a range of 1.01-7.0 Pc and a temperature within a range of 1.01-4.0 Tc. The supercritical fluid used in the present invention is a substance that easily liquefies at a comparatively low pressure and reaches the supercritical state under a low pressure and temperature. Examples of this substance include carbon dioxide, nitrogen subsulfide, sulfur hexafluoride, xenon, ethylene, ethane, chlorotrifluoromethane, and trifluoromethane. Carbon dioxide is preferably used because it is cheap and non-toxic, does not burn, has a low critical temperature, and the critical state is easily reached.

The "crystallization vessel" used in the present invention is a vessel having an inside temperature and pressure sufficient for maintaining the gas or liquid used as the supercritical fluid in either a subcritical or supercritical state. The crystallization vessel is also where diffusion and crystallization of the sample component occurs.

In the production of the crystallization product, the supercritical fluid may be mixed with a modifier when necessary. The modifier, which is also referred to as a modification agent or auxiliary solvent, when mixed with the supercritical fluid, possesses properties that change the supercritical characteristics at the critical point or nearby the critical point. The shape and size of the crystallization product may change by changing the flow rate of the modifier in relation to the flow rate of the supercritical fluid. There are no specific restrictions to the modifier used. For example, when the supercritical fluid is carbon dioxide and the sample solvent is water, alcohols such as ethanol can be used.

The sample component has a low solubility in the above supercritical fluid or mixed solution of the supercritical fluid and modifier. Therefore, the supercritical fluid and mixed solution act as a poor solvent to the sample component.

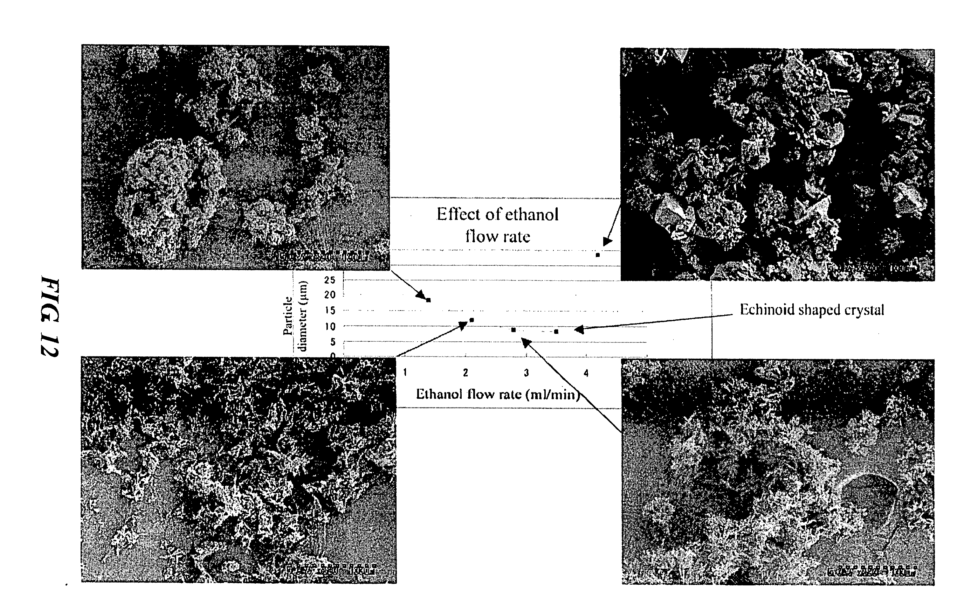

In the production of the crystallization product of the present invention, the morphology and size of the crystallization product may be related to the amount of the modifier contained in the supercritical fluid. For example, when carbon dioxide is used as a supercritical fluid, ethanol as a modifier, lactose as a sample component, and water as a solvent for dissolving the sample component, if the flow rate of the supercritical fluid is constant and the flow rate of the modifier is increased, the particle diameter gradually decreases, and when the flow rate of ethanol is 1/4 that of the carbon dioxide, the target radial spherical crystallization product is formed. If the flow rate of ethanol is increased further, the needle-shaped projections disappear, resulting in crystals with a normal shape (tomahawk-type crystals) (refer to Fig. 12). Preferable crystallization products can be experimentally obtained in other systems taking this relationship into consideration.

The following is a description of one example of an apparatus used in the production of the crystallization product of the present invention.

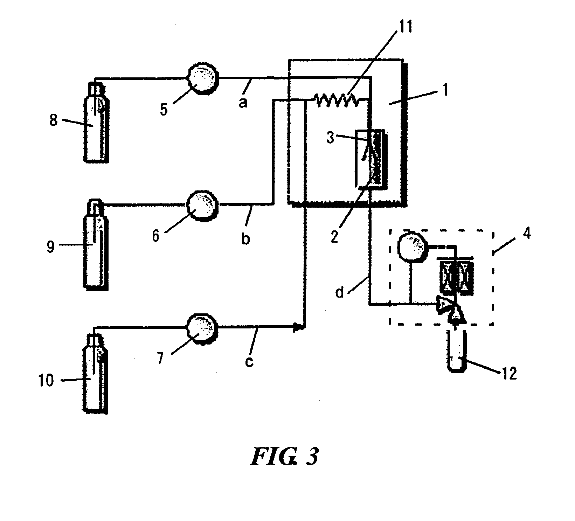

Fig. 3 is a drawing showing an outline of one example of the apparatus used in the production of the crystallization product of the present invention. In Fig., 1 represents an a column oven, 2 represents a crystallization vessel, 3 represents a nozzle, 4 represents a back pressure regulator, 5 represents a sample component solution supply pump, 6 represents a supercritical fluid supply pump, 7 represents a modifier supply pump, 8 represents a sample component solution container, 9 represents a supercritical fluid container, 10 represents a modifier container, 11 represents a mixing column (coil), and 12 represents a waste solvent recovering container. Furthermore, a represents a sample component supply pipe, b represents a supercritical fluid supply pipe, c represents a modifier supply pipe, and d represents a discharge pipe.

To produce a crystallization product using the apparatus of Fig. 3, first, the column oven 1 is set at a temperature at which a subcritical or supercritical state is maintained inside the crystallization vessel 2. Next, the supercritical fluid is supplied to the crystallization vessel 2 and the back pressure regulator 4 attached to the vessel is adjusted to a pressure under which a subcritical or supercritical state is maintained. The supercritical fluid and the modifier are passed through the supply pipes b and c, respectively, mixed in the mixing column 11, and supplied to the inside of the crystallization vessel 2. The flow rates of the supercritical fluid and modifier are adjusted to a ratio which ensures that the sample component forms the radial spherical crystallization product. For example, when carbon dioxide and ethanol are used, carbon dioxide is caused to flow at the highest flow rate and the flow rate of ethanol is adjusted to 1/4 that of the carbon dioxide. The solvent comprising the sample component suspended or dissolved therein is supplied to the crystallization vessel 2 through the sample component solution supply pipe a and enters the crystallization vessel through the nozzle 3a. Then the mixture of the supercritical fluid and modifier enters through the nozzle 3b and comes in contact with the solution of the sample component, thereby causing the sample component solution to diffuse into a mist and the sample component in the solution to crystallize inside the vessel 2. A V-shaped nozzle is used as the nozzle 3 (refer to H. Okamoto, S. Nishida, H. Todo, Y. Sakakura, K. Iida, and K. Danjo (2003): Pulmonary gene delivery by chitosan-pDNA complex powder prepared with supercritical carbon dioxide, J. Pharm. Sci. 92(2): 371-380). The crystallization product is dried and the radial spherical crystallization product is recovered from the vessel 2.

The following is believed to be the reason why a radial spherical crystallization product can be obtained by the method of the present invention. The solution comprising the sample component and the supercritical fluid or mixture of the supercritical fluid and modifier enter the crystallization vessel 2 through different flow channels without contacting each other outside the crystallization vessel 2. They come in contact with each other as soon as they are emitted into the crystallization vessel 2, and as a result, the impetus resulting from the contact causes the solution comprising the sample component to disperse (diffuse) into the crystallization vessel in a fine mist wherein the small particles of the sample component become cores forming needle-shaped projections, thereby forming spherical crystals.

### EXAMPLES

The present invention will be described in more detail with reference to Examples and Test Examples which should not be construed as limiting the present invention.

### Example 1

Using a lactose aqueous solution as the sample component solution, carbon dioxide as the supercritical fluid, ethanol as the modifier, the supercritical carbon dioxide crystallization apparatus of Fig. 3, and the equipment shown in Table 1, a radial spherical crystallization product was prepared according to the following method and evaluated.

**Table 1 Equipment**

| Equipment name | | Type | I Manufacturer |
|---|---|---|---|
| Particle distribution analyzer | | | |
| | Aerosizer | DSP 3225 | TSI |
| | Aero-Disperser | 3230 | TSI |
| Electronic chemical scale | | AX205, AT261 | Mettler Toledo |

| Supercritical carbon dioxide crystallization apparatus (Fig. 3) | | | |
|---|---|---|---|
| | Sample component supply pump | PU-1580 | JASCO |
| | Ethanol supply pump | PU-1580 | JASCO |
| | Carbon dioxide supply pump | SCF-Gel | JASCO |
| | Column oven | GC353B | GL Sciences Inc. |
| | Back pressure regulator | SCF-Bpg | JASCO |
| SEM | | S-2500 | Hitachi Ltd. |

### Crystallization method

The carbon dioxide supply pump was turned on and once the pump was cooled to -5°C, the back pressure regulator was turned on, the pressure and temperature were set, the carbon dioxide pass valve and the main stopcock of the carbon dioxide tank were opened, and the carbon dioxide supply switch was turned on to start supply of the carbon dioxide. Once the pressure reached the set value, the ethanol (modifier) supply pump was turned on to start supply of the ethanol. After the temperature and pressure inside the vessel was stationary, the sample component supply pump switch was turned on to introduce the sample component (a 20% lactose aqueous solution) into the vessel, thereby initiating crystallization. After crystallization was completed, the sample component supply and modifier supply pumps were turned off, the valves were shut, and carbon dioxide was supplied for 30 minutes or more to dry the inside of the column. Next, after turning off the carbon dioxide pump and closing the main stopcock to the carbon dioxide tank, the pressure provided by the back pressure regulator was gradually reduced. Finally, the exhaust valve was opened, the inside of the vessel was completely returned to normal temperature and pressure, and the crystallization product was collected from the inside of the column.

### Test Example 1

The aerodynamic particle diameter of the crystallization product obtained in Example 1 was measured and the morphology of the particles was observed by SEM to investigate an effect of the operating conditions of the supercritical crystallization apparatus on the particle properties.
(1) Measurement of aerodynamic particle diameter:
   In accordance with common procedure, an Aerosizer and attached PC were set up. After setting the dispersion pressure (4.0 psi), about 0.5 of a microspatula of sample was placed in the sample holder and the lid was closed, the sample holder was firmly attached, and the aerodynamic particle diameter of the sample was measured.
(2) Observation of particle formation by SEM:
   A double-sided tape was applied to a sample stand and the crystallization product was scattered onto the tape to secure the crystallization product to the sample stand, Pt-Pd vapor was deposited onto the sample stand using ion sputtering, and the particle formation was confirmed by SEM.
(3) Effect of operation conditions of the supercritical crystallization apparatus on the particle properties:

### Test conditions and method

Under the base conditions of a carbon dioxide flow rate of 14.0 ml/min, ethanol flow rate of 0.7 ml/min, sample component solution supply rate of 0.035 ml/min, pressure of 16 MPa, temperature of 35°C, and sample component concentration (lactose aqueous solution concentration) of 10% w/w, each of the operating conditions was changed in accordance with Table 2 and the particle morphology (SEM) and aerodynamic particle diameter (Aerosizer) were evaluated.

**Table 2**

| Operation | Conditions |
|---|---|
| CO₂ flow rate (ml/min) | 4.0, 6.5, 9.0, 11.5, 14.0 |
| EtOH flow rate (ml/min) | 0, 0.175, 0.350, 0.525, 0.700 |
| Pressure (MPa) | 8, 12, 16, 20, 24 |
| Temperature (°C) | 35, 40, 50, 60, 70 |
| CO₂ and EtOH flow rate (ml/min) | CO₂:4.0, 6.5, 9.0, 11.5, 14.0 |
| (The EtOH flow rate was set to 5% of the flow rate of CO₂.) | EtOH: 0.2, 0.325, 0.45, 0.575, 0.7 |

| | |
|---|---|
| * The underlined values are base conditions. | |

### Results and considerations

Each of the examination conditions, crystallization product yield, and aerodynamic diameter obtained from those conditions are summarized in Table 3.

**Table 3 Crystallization conditions, crystallization product yield, and aerodynamic 50% diameter**

| Run No. | CO₂ flow rate (ml/min) | EtOH flow rate (ml/min) | CO₂ and EtOH flow rate ratio (C/E) | Pressure (MPa) | Temperature (°C) | Yield (%) | 50% accumulation diameter (µm) |
|---|---|---|---|---|---|---|---|
| 1 | 14 | 0.7 | 20 | 16 | 35 | 45.6 | 55.62 |
| 2 | 4.0 | 0.7 | 5.7 | 16 | 35 | 63.4 | 18.33 |
| 3 | 6.5 | 0.7 | 9.3 | 16 | 35 | 32.2 | 38.96 |
| 4 | 9.0 | 0.7 | 12.9 | 16 | 35 | 34.7 | 53.29 |
| 5 | 11.5 | 0.7 | 16.4 | 16 | 35 | 34.2 | 58.19 |
| 6 | 14 | 0 | - | 16 | 35 | 0 | - |
| 7 | 14 | 0.175 | 80 | 16 | 35 | 4.8 | 42.07 |
| 8 | 14 | 0.350 | 40 | 16 | 35 | 13.0 | 35.13 |
| 9 | 14 | 0.525 | 26.7 | 16 | 35 | 7.8 | 51.16 |
| 10 | 14 | 0.7 | 20 | 8 | 35 | 52.4 | 32.59 |
| 11 | 14 | 0.7 | 20 | 12 | 35 | 38.9 | 59.99 |
| 12 | 14 | 0.7 | 20 | 20 | 35 | 32.2 | 42.66 |
| 13 | 14 | 0.7 | 20 | 24 | 35 | 19.0 | 51.54 |
| 14 | 14 | 0.7 | 20 | 16 | 40 | 22.1 | 60.90 |
| 15 | 14 | 0.7 | 20 | 16 | 50 | 24.9 | 70.87 |
| 16 | 14 | 0.7 | 20 | 16 | 60 | 22.7 | 97.73 |
| 17 | 14 | 0.7 | 20 | 16 | 70 | 26.1 | 53.66 |
| 18 | 4.0 | 0.200 | 20 | 16 | 35 | 0 | - |
| 19 | 6.5 | 0.325 | 20 | 16 | 35 | 12.7 | 39.59 |
| 20 | 9.0 | 0.450 | 20 | 16 | 35 | 28.8 | 45.06 |
| 21 | 11.5 | 0.575 | 20 | 16 | 35 | 35.5 | 58.05 |

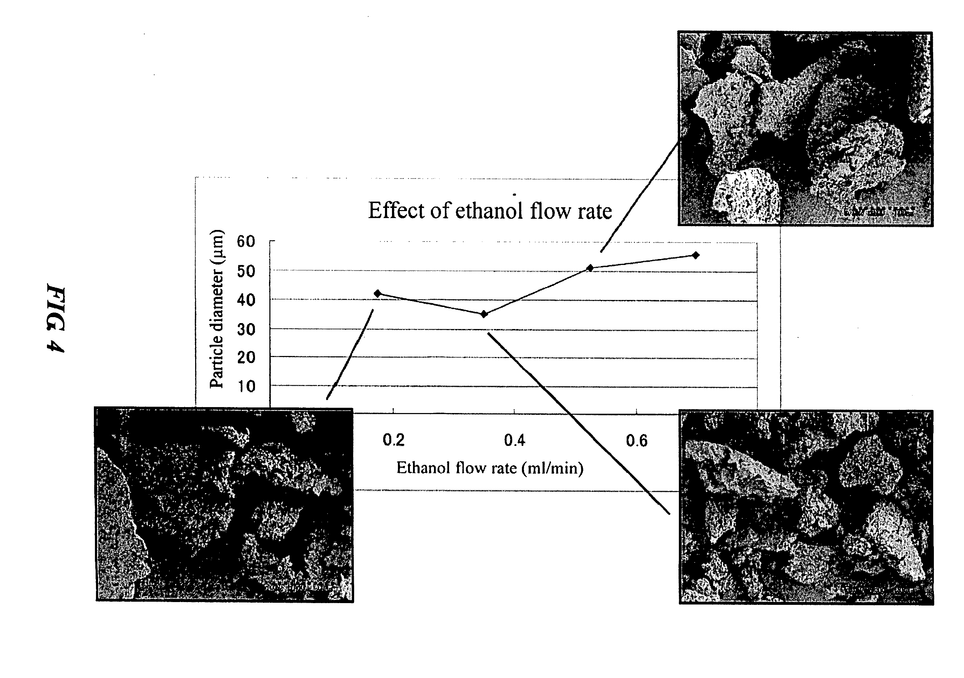

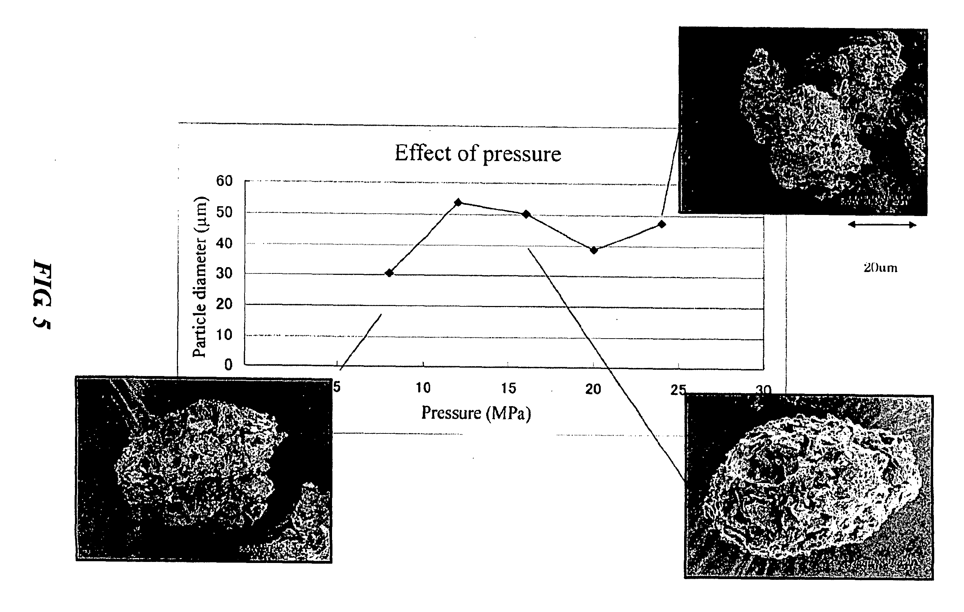

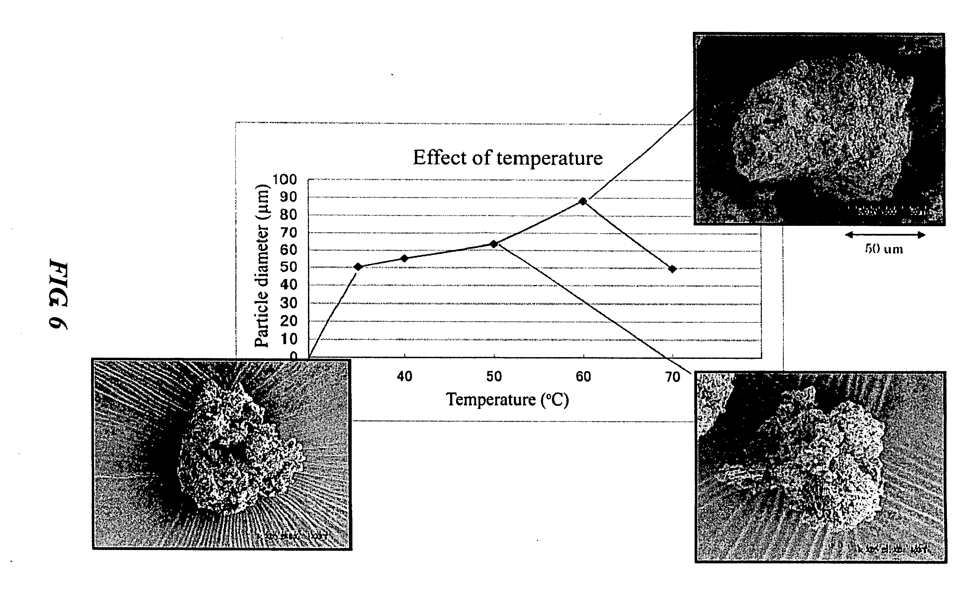

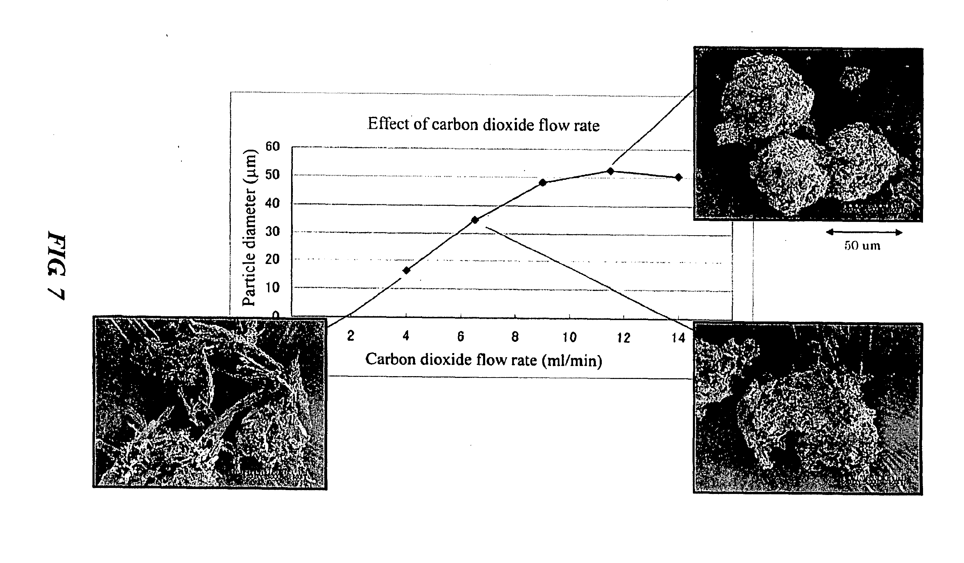

In regard to the morphology of the crystallization product, when the conditions other than the carbon dioxide flow rate were changed, the particles possessed a similar morphology when observed by SEM (refer to Figs. 4-6). On the other hand, when the carbon dioxide flow rate was changed, there was a significant change in the particle morphology (refer to Fig. 7). The change in crystallization product morphology was significant particularly when the carbon dioxide flow rate was 6.5 ml/min or less (carbon dioxide/ethanol flow rate ratio of C/E ≤ 9.3). The particle diameter also changed reflecting the change in the morphology. Specifically, the particle diameter of the needle-shaped crystals, which was as small as about 18 µm when the carbon dioxide flow rate was small, increased as the flow rate of carbon dioxide increased, resulting in massive crystallization products with a particle diameter of about 58 µm (refer to Fig. 7).

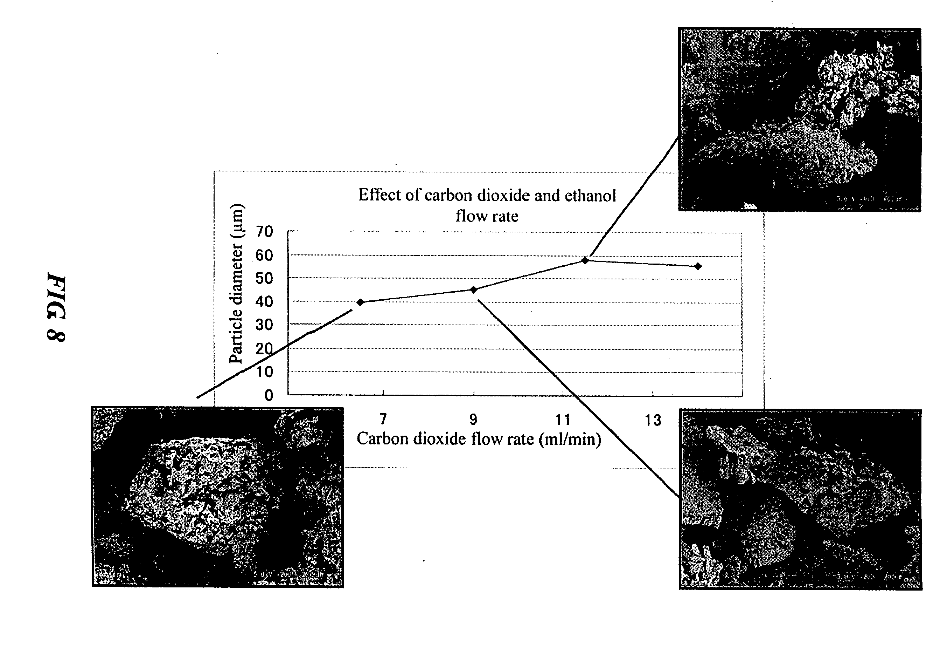

However, in the experiment in which the flow rate of ethanol was maintained at a constant 5% of the flow rate of carbon dioxide (C/E), while changing the flow rate of the mixed solvent, the change in particle morphology was small compared to when only the flow rate of the carbon dioxide was changed (refer to Fig. 8). This indicates that the flow rate ratio of carbon dioxide and ethanol (C/E) has a greater effect on the morphology of the crystallization product than the carbon dioxide flow rate alone.

Change in the ethanol flow rate alone did not greatly affect the morphology of the crystallization product in spite of the change in the C/E ratio. Specifically, a C/E ratio of 26.7 or greater was confirmed to have no effect on the particle morphology.

Taking all the above facts into consideration, it was confirmed that the morphology of the crystallization product was greatly affected when C/E ≤ 9.3.

### Test Example 2

The crystallization conditions for obtaining radial spherical crystallization products were examined based on Example 1.

### Test conditions and method

The crystallization conditions were examined in order to decrease the aerodynamic diameter of the radial spherical crystallization product obtained during examination. Under the base conditions of a carbon dioxide flow rate of 14.0 ml/min, ethanol flow rate of 3.5 ml/min, sample component solution supply rate of 0.035 ml/min, pressure of 25 MPa, temperature of 35°C, and sample component concentration (lactose aqueous solution concentration) of 20% w/w, each of the operating conditions was changed in accordance with Table 4 and the particle morphology (SEM) and aerodynamic particle diameter (Aerosizer) were evaluated. However, in the experiment wherein the ethanol flow rate was changed, conditions of a sample component concentration of 10% w/w and pressure of 30 MPa were used.

**Table 4 Conditions of examination of the radial spherical crystallization product**

| Operation | Conditions |
|---|---|
| EtOH flow rate (ml/min)* | 1.4, 2.1, 2.8, 3.5, 4.2 |
| Sample component solution flow rate (ml/min) | 0.010, 0.020, 0.035, 0.050 |
| Pressure (MPa) | 10, 15, 20, 25 |
| Temperature (°C) | 35, 50, 60, 70 |

| | |
|---|---|
| * A pressure of 30 MPa was used only in the experiment wherein the EtOH rate was changed. | |
| ** The underlined values are base conditions. | |

### Results and considerations

The examination conditions, crystallization product yield, and aerodynamic diameter obtained from those conditions are summarized in Table 5.

**Table 5 Crystallization conditions, crystallization product yield, and aerodynamic 50% diameter**

| Run No. | EtOH flow rate (ml/min) | CO₂ and EtOH flow rate ratio (C/E) | Sample component solution flow rate (ml/min) | Pressure (MPa) | Temperature (°C) | Yield (%) | 50% accumulation diameter (µm) |
|---|---|---|---|---|---|---|---|
| 22 | 3.5 | 4 | 0.020 | 25 | 35 | 77.8 | 6.65 |
| 23* | 1.4 | 10 | 0.035 | 30 | 35 | 80.3 | 18.00 |
| 24* | 2.1 | 6.7 | 0.035 | 30 | 35 | 86.9 | 12.06 |
| 25* | 2.8 | 5 | 0.035 | 30 | 35 | 81.1 | 8.91 |
| 26* | 3.5 | 4 | 0.035 | 30 | 35 | 73.9 | 8.26 |
| 27* | 4.2 | 3.3 | 0.035 | 30 | 35 | 94.1 | 33.72 |
| 28 | 3.5 | 4 | 0.010 | 25 | 35 | 60.1 | 7.30 |
| 29 | 3.5 | 4 | 0.035 | 25 | 35 | 78.1 | 7.07 |
| 30 | 3.5 | 4 | 0.050 | 25 | 35 | 75.5 | 7.03 |
| 31 | 3.5 | 4 | 0.020 | 10 | 35 | 74.2 | 7.29 |
| 32 | 3.5 | 4 | 0.020 | 15 | 35 | 56.9 | 7.62 |
| 33 | 3.5 | 4 | 0.020 | 20 | 35 | 62.5 | 8.45 |
| 34 | 3.5 | 4 | 0.020 | 25 | 50 | 66.9 | 5.24 |
| 35 | 3.5 | 4 | 0.020 | 25 | 60 | 65.3 | 6.96 |
| 36 | 3.5 | 4 | 0.020 | 25 | 70 | 64.5 | 13.76 |

| | | | | | | | |
|---|---|---|---|---|---|---|---|
| * Only these experiments were conducted using a sample component solution concentration of 10% w/w, sample component solution supply rate of 0.035 ml/min, and pressure of 30 MPa. | | | | | | | |

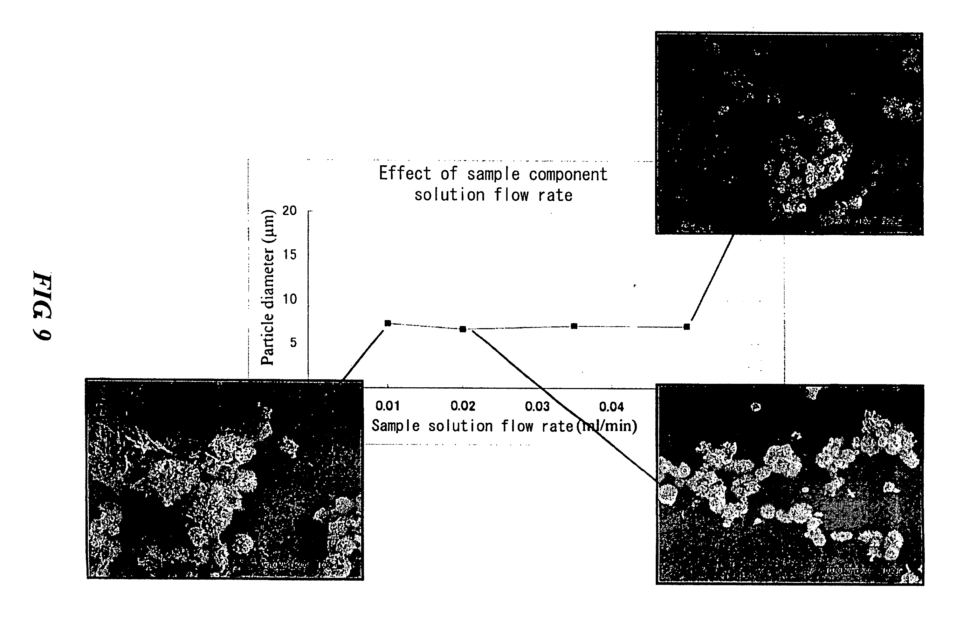

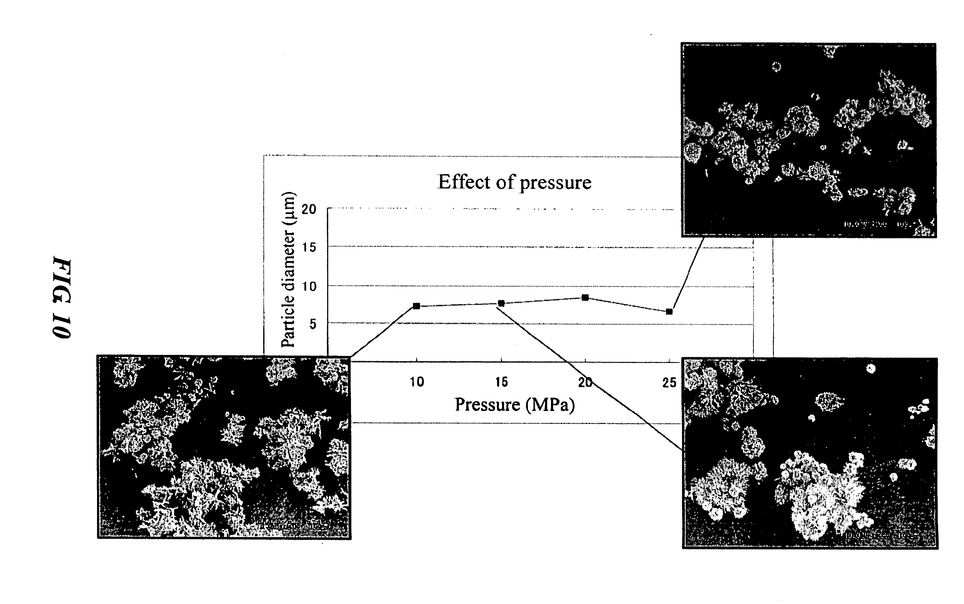

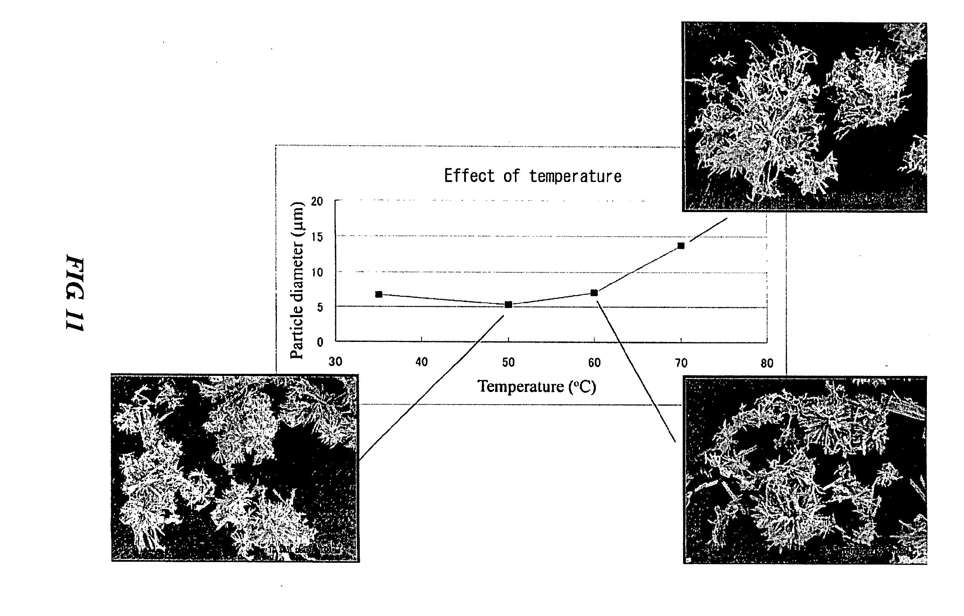

With regard to the morphology of the crystallization product, the results show that changes in the conditions other than the ethanol flow rate did not dramatically change the morphology of the radial spherical crystallization product (refer to Figs. 9-11).

On the other hand, when the ethanol flow rate was changed, the morphology of the product dramatically changed from a needle-shaped crystal aggregate to a needle-shaped crystallization product to a radial spherical crystallization product and finally to a mixed crystallization product comprising tomahawk type and radial spherical crystals as the flow rate of ethanol was increased (refer to Fig. 12). Particle diameter, in the same manner as the morphology, changed greatly in a range of about 7-34 µm when the ethanol flow rate was changed. In regard to the temperature change, although a slight change in particle diameter was observed, there was almost no change in the morphology of the crystallization product. For this reason, a particle diameter change in the range of about 5-14 µm was confirmed, which is not as great as the change resulting from the ethanol flow rate change. In regard to the other crystallization conditions, almost no change was observed in the morphology and the particle diameter remained in a range of about 6-8 µm, also exhibiting almost no change.

Based on the dramatic change in the morphology caused by the change in the ethanol flow rate, as discussed in the above Test Example 1, change in the crystallization morphology is greatly influenced by the flow rate ratio of carbon dioxide to ethanol (C/E). The smallest aerodynamic 50% accumulation diameter, which was 5.2 µm, was seen in Run No. 34.

### Crystallization conditions and morphology

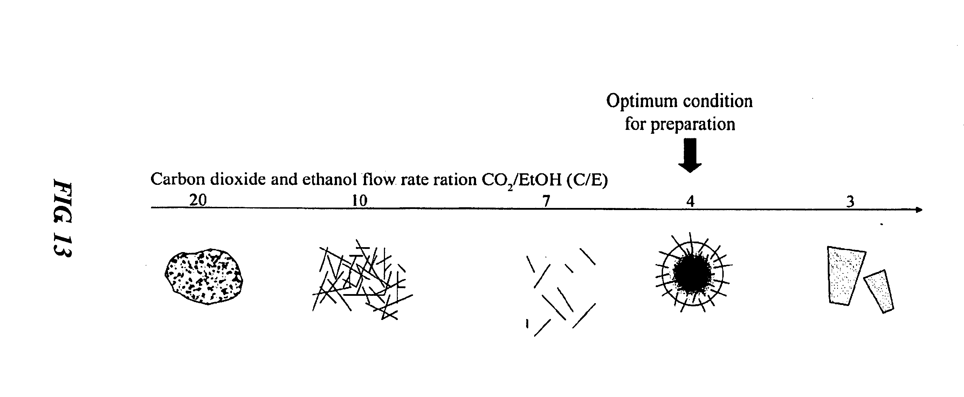

As the results of the Test Example 1 and Test Example 2, the flow rate ratio of carbon dioxide to ethanol (C/E) mostly dominates the morphology of the crystallization product irrespective of the change in temperature and pressure. The C/E relationship is shown in Fig. 13. Production of the radial spherical crystallization product was possible when the C/E is 4.

As a result of examining the effects of changes in the supercritical carbon dioxide crystallization operation conditions on the crystallization of particles using lactose as a sample component, it was confirmed that the carbon dioxide/ethanol flow rate difference (C/E) had a great effect on the particle morphology and diameter.

As a result of examining crystallization conditions to reduce particle diameter in order to provide a radial spherical crystallization product useful as a DPI carrier for delivering a drug to the lungs, a crystallization product with an aerodynamic 50% accumulation diameter of 5.2 µm was obtained.

### Example 2

### DPI preparation:

A salbutamol sulfate DPI preparation was prepared in the following manner and the *in vitro* inhaling characteristics thereof were evaluated. As a comparative product, a DPI prepared using commercially available lactose was used. The results are shown in Table 6.

### Manufacturing method

Salbutamol sulfate and a radial spherical crystallization product (obtained in Run No. 22, shown in Table 5), both sieved at 250M, were mixed at a ratio of 3:7 to obtain the preparation of the present invention (Preparation 1 of the present invention). As a result of six quantitative assays, the preparation was determined to comprise a homogenous mixture with a 29.2% content and RSD of 3.6%. Salbutamol sulfate and a commercially available lactose LH200 (manufactured by Borculo Domo Ingredients), both sieved at 250M, were mixed at a ratio of 3:7 to obtain the comparative product (Comparative Product 1). As a result of six quantitative assays, the preparation was determined to comprise a homogenous mixture with a 28.8% content and RSD of 1.6%.

### Inhalation characteristics evaluation test

3 mg each of Preparation 1 of the present invention and the Comparative Product 1 were filled into gelatin capsules. An e-haler was used as the inhalation device. The e-haler was attached to an Andersen cascade impactor (ACI) through a mouthpiece, and the contents were inhaled for six seconds at a rate of 40.01/min using a vacuum pump. The preparations was puffed twice (two 3 mg capsules) per measurement. After inhaling, the sample deposited on each fraction of stages 0-7, throat, and the device were washed out into a measuring flask using a solvent to obtain sample solvents. Using the HPLC method, the amount of salbutamol sulfate of the preparation deposited on each fraction was assayed to determine the ratio of the amount of preparation transported to the lung to the amount filled in the capsule. The measurement was repeated twice for each preparation. The results are shown in Table 6.

**Table 6**

| | n | Ratio of the amount of preparation transported to the lung to the amount filled in the capsule | Average |
|---|---|---|---|
| Preparation 1 of the present invention | 1 | 35.5 | 33.9 |
| | 2 | 32.3 | |
| Comparative preparation 1 | 1 | 9.9 | 10.4 |
| | 2 | 10.8 | |

### Example 3

A radial spherical crystallization product was produced by the following crystallization method using a 20% salbutamol sulfate aqueous solution as a sample component solution, carbon dioxide as a supercritical fluid, ethanol as a modifier, and the supercritical carbon dioxide crystallization appartus of Fig. 3. The obtained crystallization product was evaluated by the following method. The devices used in Table 1 of Example 1 were used in the production and evaluation.

### Crystallization method

A radial spherical crystallization product of salbutamol sulfate was obtained as a white powder in the same manner as in Example 1 except for using a 20% salbutamol sulfate aqueous solution as a sample component solution. The crystallization conditions are shown in Table 7.

**Table 7**

| Operation | Conditions |
|---|---|
| CO₂ flow rate (ml/min) | 14 |
| EtOH flow rate (ml/min) | 2.8 |
| Sample component solution flow rate (ml/min) | 0.020 |
| Pressure (MPa) | 25 |
| Temperature (°C) | 35 |

### Evaluation of the crystallization product

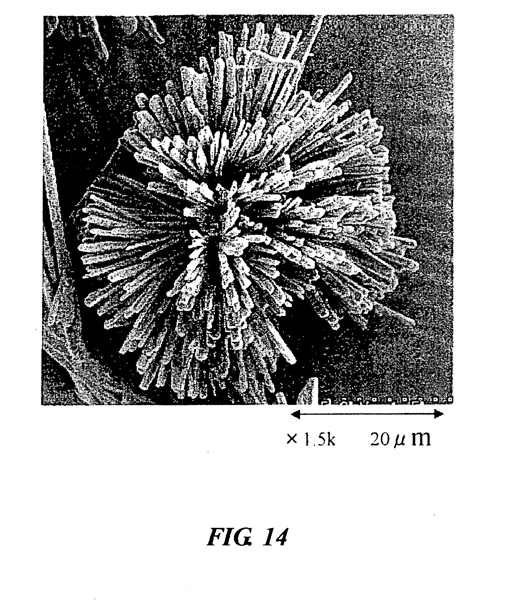

In the same manner as (2) in Test Example 1, the particle morphology was observed by SEM. The results are shown in Fig. 14.

### Industrial Applicability

Various applications of the radial spherical crystallization product of the present invention obtained in the above-described manner can be considered. One example is a drug such as a DPI preparation. Specifically, a drug incorporated into a radial spherical crystallization product in accordance with the present invention can be delivered to the pulmonary alveoli or bronchial tubes by inhalation or the like.

In addition, if the radial spherical crystallization product is used as a drug carrier according to the present invention, a drug with a higher content, for example, 30% or more, of drug substances than in conventional DPI carriers can be obtained, resulting in excellent *in vitro* inhalation characteristics.

### Brief Description of the Drawings

Fig. 1 is a scanning electron micrograph of a radial spherical crystallization product (lactose).
Fig. 2 is a scanning electron micrograph of a radial spherical crystallization product (lactose).
Fig. 3 is a schematic diagram of a supercritical fluid crystallization apparatus.
Fig. 4 is a graph showing the effects of ethanol flow rate on change in particle diameter and morphology.
Fig. 5 is a graph showing the effects of pressure on change in particle diameter and morphology.
Fig. 6 is a graph showing the effects of temperature on change in particle diameter and morphology.
Fig. 7 is a graph showing the effects of carbon dioxide flow rate on change in particle diameter and morphology.
Fig. 8 is a graph showing the effects of the ratio of ethanol flow rate to carbon dioxide flow rate (5%) on change in particle diameter and morphology.
Fig. 9 is a graph showing the effects of sample component solution flow rate on change in particle diameter and morphology.
Fig. 10 is a graph showing the effects of pressure on change in particle diameter and morphology.
Fig. 11 is a graph showing the effects of temperature on change in particle diameter and morphology.
Fig. 12 is a graph showing the effects of ethanol flow rate on change in particle diameter and morphology.
Fig. 13 is a simulated diagram showing the relationship between the particle morphology of the crystallization product and the ratio of carbon dioxide flow rate to ethanol flow rate (C/E).
Fig. 14 is a scanning electron micrograph of a radial spherical crystallization product (salbutamol sulfate).

### Explanation of Symbols

- 1: Column oven
- 2: Crystallization vessel
- 3: Nozzle
- 4: Back pressure regulator
- 5: Sample component solution supply pump
- 6: Supercritical fluid supply pump
- 7: Modifier supply pump
- 8: Sample component solution container
- 9: Supercritical fluid container
- 10: Modifier container
- 11: Mixing column (coil)
- 12: Waste solvent recovering container
- a: Sample component supply pipe
- b: Supercritical fluid supply pipe
- c: Modifier supply pipe
- d: Discharge pipe

## Claims

1. A radial spherical crystallization product comprising needle-shaped projections radiating from the crystal core.

2. The radial spherical crystallization product according to claim 1 having an aerodynamic diameter of 0.1-20 µm.

3. The radial spherical crystallization product according to claim 1 or 2 having a bulk density of 100 mg/ml or less.

4. A radial spherical crystallization product obtained by emitting a supercritical fluid or a mixture of a supercritical fluid and a modifier and a solution comprising a sample component into a crystallization vessel through different flow channels to cause them to come in contact with each other as they are emitted into the crystallization vessel.

5. The radial spherical crystallization product according to claim 4, wherein the supercritical fluid or the mixture of the supercritical fluid and a modifier is a poor solvent for the sample component.

6. The radial spherical crystallization product according to claim 4 or 5, wherein the sample component is a pharmaceutical drug.

7. The radial spherical crystallization product according to claim 4 or 5, wherein the sample component is a drug carrier.

8. The radial spherical crystallization product according to claim 7, wherein the drug carrier is a sugar or sugar alcohol.

9. The radial spherical crystallization product according to any one of claims 4-8, wherein the supercritical fluid is carbon dioxide.

10. The radial spherical crystallization product according to any one of claims 4-9, wherein the modifier is ethanol.

11. The radial spherical crystallization product according to any one of claims 1-10 used as a raw drug for a dry powder preparation.

12. The radial spherical crystallization product according to any one of claims 1-10 used as a drug carrier for a dry powder inhaler.

13. A method for manufacturing a radial spherical crystallization product **characterized by** injecting a supercritical fluid or a mixture of a supercritical fluid and a modifier and a solution comprising a sample component into a crystallization vessel through different flow channels to cause them to come in contact with each other as they are emitted into the crystallization vessel.

14. The method for manufacturing a radial spherical crystallization product according to claim 13, wherein the supercritical fluid or the mixture of the supercritical fluid and a modifier is a poor solvent for the sample component.

15. The method for manufacturing a radial spherical crystallization product according to claim 13 or 14, wherein the sample component is a pharmaceutical drug.

16. The method for manufacturing a radial spherical crystallization product according to claim 13 or 14, wherein the sample component is a drug carrier.

17. The method for manufacturing a radial spherical crystallization product according to claim 16, wherein the drug carrier is a sugar or sugar alcohol.

18. The method for manufacturing a radial spherical crystallization product according to any one of claims 13-17, wherein the supercritical fluid is carbon dioxide.

19. The method for manufacturing a radial spherical crystallization product according to any one of claims 13-18, wherein the modifier is ethanol.

20. A dry powder inhaler comprising the radial spherical crystallization product according to claim 6 as an active ingredient.

21. A dry powder inhaler comprising the radial spherical crystallization product according to claim 7 or 8 as a carrier.
